(19)

Europäisches Patentamt

European Patent Office

Office européen des brevets

(11) **EP 4 558 201 B1**

(12) **EUROPEAN PATENT SPECIFICATION**

(45) Date of publication and mention
of the grant of the patent:
**29.04.2026  Bulletin 2026/18**

(21) Application number: **23754534.8**

(22) Date of filing: **19.07.2023**

(51) International Patent Classification (IPC):
**A61M 13/00** *(2006.01)*

(52) Cooperative Patent Classification (CPC):
**A61M 13/006;** A61M 16/202; A61M 16/203;
A61M 2016/0027; A61M 2016/1025;
A61M 2202/0208; A61M 2202/0225; A61M 2205/07;
A61M 2205/3306; A61M 2205/3331;
A61M 2205/3372; A61M 2205/50

(86) International application number:
**PCT/US2023/028107**

(87) International publication number:
**WO 2024/020073 (25.01.2024 Gazette 2024/04)**

(54) **SYSTEM FOR CONTROLLING INSUFFLATION GAS CONCENTRATION USING AN OPTICAL OXYGEN SENSOR**

SYSTEM ZUR STEUERUNG DER INSUFFLATIONSGASKONZENTRATION UNTER VERWENDUNG EINES OPTISCHEN SAUERSTOFFSENSORS

SYSTÈME DE RÉGULATION DE LA CONCENTRATION DE GAZ D'INSUFFLATION À L'AIDE D'UN CAPTEUR OPTIQUE D'OXYGÈNE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority:  **21.07.2022   US 202263391018 P**

(43) Date of publication of application:
**28.05.2025   Bulletin 2025/22**

(73) Proprietor: **CONMED Corporation**
**Largo, FL 33773 (US)**

(72) Inventors:
• **KOLTZ, Michael L., Jr.**
**Aurora, CO 80016 (US)**

• **SELL, Leo**
**Centennial, CO 80111 (US)**
• **TADESSE, Nebiyu**
**Aurora, CO 80015 (US)**

(74) Representative: **Hoefer & Partner Patentanwälte mbB**
**Pilgersheimer Straße 20**
**81543 München (DE)**

(56) References cited:
| | |
|---|---|
| WO-A1-2018/039239 | US-A1- 2014 336 567 |
| US-A1- 2018 221 598 | US-A1- 2019 365 414 |
| US-A1- 2022 117 629 | US-B2- 9 950 127 |

**Description**

## BACKGROUND OF THE DISCLOSURE

### 1. Field of the Disclosure

The subject disclosure is directed to endoscopic surgery, and more particularly, to a surgical gas delivery system that includes an optical oxygen sensor for use in controlling insufflation gas concentration in a gas recirculation circuit.

### 2. Description of Related Art

Laparoscopic or "minimally invasive" surgical techniques are becoming commonplace in the performance of procedures such as cholecystectomies, appendectomies, hernia repair and nephrectomies. Such procedures commonly involve filling or "insufflating" the abdominal cavity with a pressurized fluid, such as carbon dioxide, to create an operating space, which is referred to as a pneumoperitoneum.

The documents US 2022/117629 A1, US 2019/365414 A1, US 2014336567 A1, WO 2018/039239 A1 and US 2018/221598 A1 disclose surgical gas delivery systems according to the state of the art.

Insufflators are used to deliver pressurized insufflation gas to a patient's abdominal cavity during a surgical procedure, including insufflators those that are adapted and configured to recirculate insufflation gas through a recirculation circuit as disclosed in commonly assigned U.S. Patent No. 9,950,127.

Insufflators, such as the device disclosed in U.S. Patent No. 9,950,127, employ metal oxide film oxygen sensors to monitor insufflation gas concentration. These sensors tend to require the film to be heated to several hundred degrees to obtain a useful semiconducting signal. The need to heat the film requires the sensor system to inherit a number of unfavorable characteristics. First, high power consumption, which requires large interconnect, large power supplies, and the means for removing the heat losses of these components. Additionally, the metal oxide film sensors need to be made of heat resistant materials, which are heavier and more expensive than polymer based optical sensor.

Also, metal oxide film sensors typically have a sintered metal filter which frequently becomes blocked by corrosion or particulate such as compressor piston seal wear debris. Blocked sensor filters require frequent service, reducing insufflator system availability. Furthermore, metal oxide film sensors require circuitry to monitor and control the sensor temperature, adding to system complexity, size, and expense. Finally, metal oxide film sensors require a period to heat the film to operating temperature. This time can be 60 seconds or greater, which can interrupt operating room workflow. All of these negative characteristics challenge the needs of the modern operating room.

Comparatively, optical or infra-red oxygen sensors require no heating and have total power consumption much less than 1 Watt compared to the multi-Watt power consumption of the metal oxide film sensors. The functional elements of the infra-red oxygen sensor comprise an infra-red emitter, and infra-red detector, a phosphor impregnated film, an amplifier, and an I2C communication circuit. These components, with the exception of the film, are low cost and commercially available from numerous sources.

For these reasons, it would be beneficial to employ an optical oxygen sensor to monitor insufflation gas concentration in the gas recirculation circuit of a surgical gas delivery device.

## SUMMARY OF THE DISCLOSURE

The invention is defined in the appended independent claim 1. Preferred embodiments are matter of the dependent claims.

The subject disclosure is directed to a new and useful surgical gas delivery system that includes a gas recirculation circuit for insufflation gas, an optical oxygen sensor, a gas metering valve for metering a flow of insufflation gas from a gas source into the gas recirculation circuit, and a processor for controlling the gas metering valve based at least in part upon signals received from the optical oxygen sensor and a pressure sensor to adjust insufflation gas concentration within the gas recirculation circuit.

A pump is operatively associated with the gas recirculation circuit to recirculate insufflation gas therethrough. The gas metering valve can be a solenoid valve or a proportional valve. The pressure sensor is adapted and configured to measure insufflation gas pressure within the gas recirculation circuit, and the optical oxygen sensor is adapted and configured to quantify a partial pressure of oxygen in the gas recirculation circuit. This is done by measuring an infra-red wavelength of light absorption from a phosphor containing element and converting that measurement to a digital signal. Because this type of pressure sensor is sensitive to temperature changes, temperature sensors are incorporated into the oxygen sensor package to continuously monitor temperature so temperature changes can be considered and compensated for during processing.

The subject disclosure further comprise to a system for adjusting insufflation gas concentration within a gas recirculation circuit, which includes a gas metering valve for metering a flow of insufflation gas from a gas source into the gas recirculation circuit, an optical oxygen sensor configured to optically quantify a partial pressure of oxygen in the gas recirculation circuit, a pressure sensor for measuring insufflation gas pressure in the gas recirculation circuit, and a processor for controlling the gas metering valve based at least in part upon the partial pressure of oxygen in the gas recirculation circuit and the insufflation gas pressure in the gas recirculation circuit signals to adjust insufflation gas concentration within the gas recirculation circuit.

These and other features of the system and method of the subject disclosure will become more readily apparent from the following detailed description of the disclosed embodiments taken in conjunction with the drawings.

## BRIEF DESCRIPTION OF THE DRAWINGS

So that those skilled in the art will readily understand how to make and use the gas delivery system of the subject disclosure without undue experimentation, embodiments thereof will be described in detail herein below with reference to the figures wherein:

Fig. 1 is a high level flow diagram of the surgical gas delivery system of the subject disclosure, which includes a gas recirculation circuit;

Fig. 2 is a pneumatic schematic of the surgical gas delivery device of the subject disclosure, which includes the gas recirculation circuit of Fig. 1; and

Fig. 3 is a perspective view of a gaseous sealing manifold assembly within the surgical gas delivery device of Fig. 2, which includes, among other components and things, a gas metering valve, an optical oxygen sensor and a pressure sensor for use in controlling insufflation gas concentration in the gas recirculation circuit.

## DETAILED DESCRIPTION OF THE EMBODIMENTS

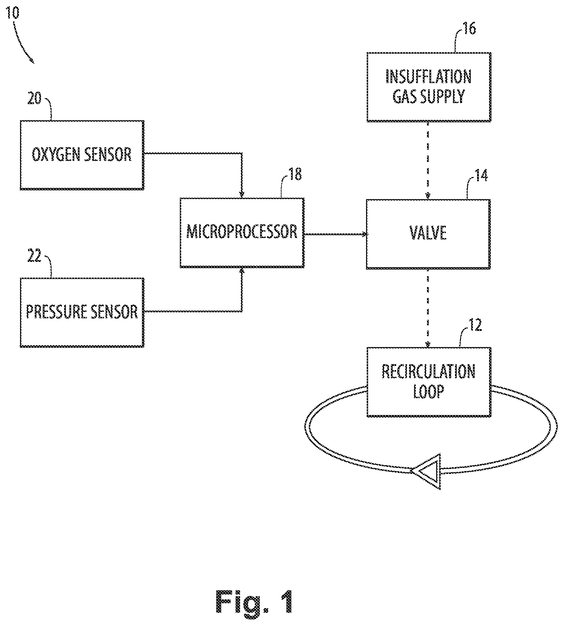

Referring now to the drawings, wherein like reference numerals identify similar features or aspects of the subject disclosure, there is illustrated in Fig. 1 a new and useful surgical gas delivery system configured in accordance with the subject disclosure and designated generally by reference numeral 10. Gas delivery system 10 is of the multi-modal type disclosed for example in commonly assigned U.S. Patent No. 9,950,127.

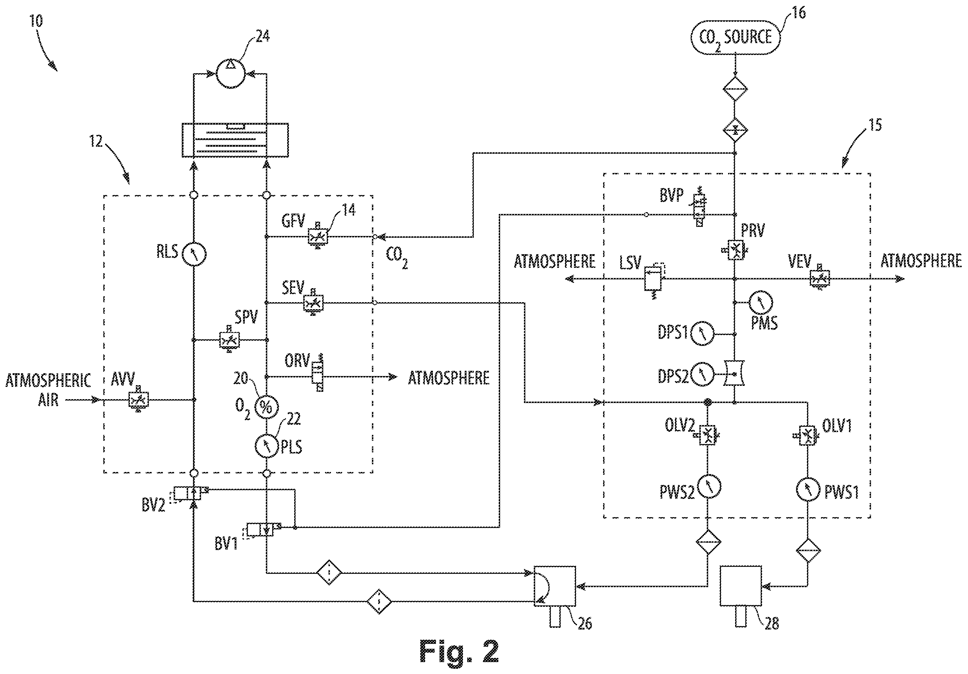

Gas delivery system 10 includes a gas recirculation circuit 12 for insufflation gas, a gas metering valve 14 (i.e., GFV in Fig. 2) for metering a flow of insufflation gas from an insufflation gas supply source 16 (e.g. a portable gas bottle or tank) into the gas recirculation circuit 12, and a processor 18 running a valve control algorithm for controlling the gas metering valve 14. More particularly, the processor 18 controls the gas metering valve 14 based upon signals received from an optical oxygen sensor 20 and a pressure sensor 22 (i.e., PLS in Fig. 2) to adjust insufflation gas concentration within the gas recirculation circuit 12.

Referring now to Fig. 2, there is illustrated a more detailed pneumatic schematic of the gas delivery system 10 of the subject disclosure, which includes the gas recirculation circuit 12 and a cooperating insufflation circuit 15. As shown, a compressor or pump 24 is operatively associated with the gas recirculation circuit 12 to recirculate insufflation gas therethrough. More particularly, the gas recirculation circuit 12 circulates a flow of pressurized insufflation gas through a gas sealed surgical access device or trocar 26, which is operatively connected to the gas delivery device 10 by way of a filtered tube set. The surgical access port 26 is in direct communication with a patient's body cavity. An example of a gas sealed access port is disclosed in commonly assigned U.S. Patent No. 8,795,223. An example of a filtered tube set is disclosed in commonly assigned U.S. Patent No. 9,067,030, which is incorporated herein by reference. A valve sealed trocar 28 cooperates with the insufflation circuit 15. The insufflation circuit 15 also communicates with the gas sealed access device 26.

It is envisioned that the gas metering valve 14 (i.e., GFV in Fig. 2), which meters insufflation gas into the gas recirculation circuit 12, can be an on-off solenoid valve or a proportional valve for facilitating more precise dynamic flow control, depending upon the application and availability of components. The pressure sensor 22 (i.e., PLS in Fig. 2) is adapted and configured to measure insufflation gas pressure within the gas recirculation circuit 12. It is located on the high pressure or discharge side of pump 24, downstream from the oxygen sensor 20 and upstream from the access port 26.

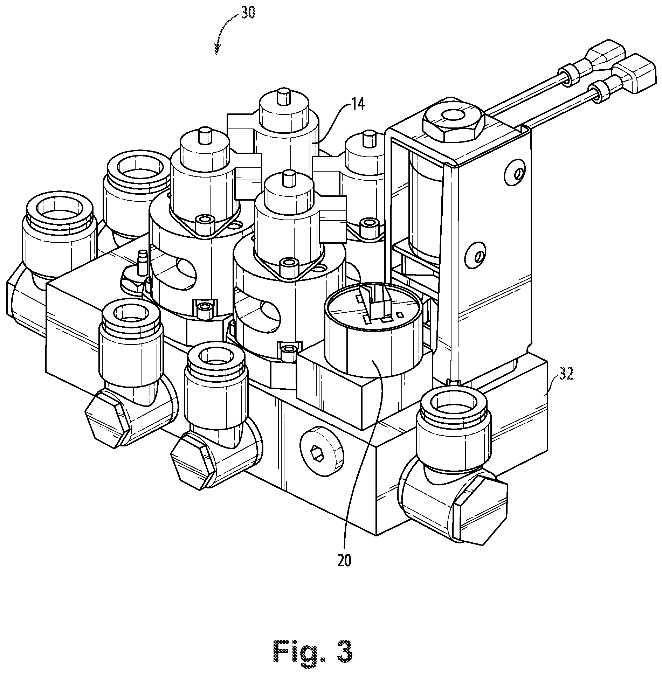

Referring now to Fig. 3, the gas metering valve 14 and the oxygen sensor 20 are components of a gaseous sealing manifold assembly 30, which is housed within the surgical gas delivery system 10. (See e.g., commonly assigned U.S. Patent Application Publication 2022/233794). The gaseous sealing manifold 30 is a modular and replaceable unit defined by a compact manifold body 32 that has an internal communication network of interconnected passages, which facilitate the flow of surgical gas and air between and among the various components of the gas recirculation circuit 12. The manifold assembly 30, along with the pump 24 and the gas sealed access port 26, form the recirculation circuit 12 of gas delivery system 10.

The optical oxygen sensor 20, unlike the gas quality sensors in the prior art, is adapted and configured to quantify a partial pressure of oxygen in the gas recirculation circuit 12. This is done optically by measuring an infra-red wavelength of light absorption from a phosphor containing element and converting that measurement to a digital signal.

An optical oxygen concentration sensor of this type is commercially available as Item FDO2 from Pyro Science GmbH of Aachen, Germany. Because this type of sensor is sensitive to temperature changes, temperature sensors are incorporated into the oxygen sensor package to continuously monitor temperature so temperature changes can be considered and compensated for during processing, as described in U.S. Patent No. 10,495,619.

The oxygen sensor 20 infers insufflation gas, usually carbon dioxide, concentration by assuming: (1) that the recirculating gas in circuit 12 is comprised exclusively of insufflation gas and air; and (2) that ambient air contains a specific percentage of oxygen, usually 20% or one-fifth. By sensing oxygen versus the insufflation gas, the device is not limited to a single insufflation gas.

In accordance with the subject disclosure, insufflation gas concentration in the gas recirculation circuit is calculated by way of a computer-implemented method using signals from oxygen sensor 20 and pressure sensor 22 and the assumptions stated above as follows:

$$CONC\_IG = 1 - \left[ \frac{PPO2}{PRESS \cdot CONC\_O2} \right]$$

Where:

CONC_IG: Insufflation Gas Concentration
PPO2: Partial Pressure of Oxygen (Signal from Optical Sensor 20)
PRESS: Pressure of Gas at Sensor (Signal from Pressure Sensor 22)
CONC_O2: Assumed Concentration of Oxygen in Ambient Air

Using these calculations, the processor 18 can command or otherwise control the gas metering valve 14 to adjust the insufflation gas concentration within the gas recirculation circuit 12. More particularly, a PID (proportional-integral-derivative) control loop controls the insufflation gas concentration as the pressurized gas is recirculating through the circuit 12, while the pump/compressor 24 is running.

As will be appreciated by those skilled in the art, aspects of the present disclosure may be embodied as a system, method or computer program product. Accordingly, aspects of this disclosure may take the form of an entirely hardware embodiment, an entirely software embodiment (including firmware, resident software, microcode, etc.), or an embodiment combining software and hardware aspects, all possibilities of which can be referred to herein as a "circuit," "module," or "system." A "circuit," "module," or "system" can include one or more portions of one or more separate physical hardware and/or software components that can together perform the disclosed function of the "circuit," "module," or "system", or a "circuit," "module," or "system" can be a single self-contained unit (e.g., of hardware and/or software). Furthermore, aspects of this disclosure may take the form of a computer program product embodied in one or more computer readable medium(s) having computer readable program code embodied thereon.

Any combination of one or more computer readable medium(s) may be utilized. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer readable storage medium may be any tangible medium that can contain, or store a program for use by or in connection with an instruction execution system, apparatus, or device.

A computer readable signal medium may include a propagated data signal with computer readable program code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

Program code embodied on a computer readable medium may be transmitted using any appropriate medium, including but not limited to wireless, wireline, optical fiber cable, RF, etc., or any suitable combination of the foregoing.

Computer program code for carrying out operations for aspects of this disclosure may be written in any combination of one or more programming languages, including an object oriented programming language such as Java, Smalltalk, C++ or the like and conventional procedural programming languages, such as the "C" programming language or similar programming languages. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of this disclosure may be described above with reference to flowchart illustrations and/or block diagrams of methods, apparatus (systems) and computer program products according to embodiments of this disclosure. It will be understood that each block of any flowchart illustrations and/or block diagrams, and combinations of blocks in any flowchart illustrations and/or block diagrams, can be implemented by computer program instructions. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in any flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified herein.

While the subject disclosure has been shown and described with reference to certain embodiments, those skilled in the art will readily appreciate that changes and/or modifications may be made thereto without departing from the scope of the subject disclosure.

**Claims**

1. A surgical gas delivery system (10) comprising:

    a) a gas recirculation circuit (12) for insufflation gas;
    b) an optical oxygen sensor (20) for monitoring insufflation gas concentration;
    c) a gas metering valve (14) for metering a flow of insufflation gas from a gas source (16) into the gas recirculation circuit (12); and
    d) a processor (18) for controlling the gas metering valve (14) based at least in part upon signals received from the optical oxygen sensor (20) and a pressure sensor (22) to adjust insufflation gas concentration within the gas recirculation circuit (12).

2. A surgical gas delivery system (10) as recited in Claim 1, a pump (24) is operatively associated with the gas recirculation circuit (12) to recirculate insufflation gas therethrough.

3. A surgical gas delivery system (10) as recited in Claim 1, wherein the gas metering valve (14) is a solenoid valve.

4. A surgical gas delivery system (10) as recited in Claim 1, wherein the gas metering valve (14) is a proportional valve.

5. A surgical gas delivery system (10) as recited in Claim 1, wherein the pressure sensor (22) is adapted and configured to measure insufflation gas pressure within the gas recirculation circuit (12).

6. A surgical gas delivery system (10) as recited in Claim 1, wherein the optical oxygen sensor (20) is adapted and configured to quantify a partial pressure of oxygen in the gas recirculation circuit (12).

7. A surgical gas delivery system (10) as recited in Claim 1, wherein the optical oxygen sensor (20) is adapted and configured to quantify a partial pressure of oxygen in the gas recirculation circuit (12) by measuring an infra-red wavelength of light absorption from a phosphor containing element and converting that measurement to a digital signal.

8. A surgical gas delivery system (10) as recited in Claim 1, comprising a system for adjusting insufflation gas concentration within the gas recirculation circuit (12), wherein said system for adjusting insufflation gas concentration within the gas recirculation circuit (12) comprises:

    b) the optical oxygen sensor (20) being configured to optically quantify a partial pressure of oxygen in the gas recirculation circuit (12);
    c) the pressure sensor (22) being suitable for measuring insufflation gas pressure in the gas recirculation circuit (12); and
    d) the processor (18) being suitable for controlling the gas metering valve (14) based at least in part upon the partial pressure of oxygen in the gas recirculation circuit (12) and the insufflation gas pressure in the gas recirculation circuit (12) signals to adjust insufflation gas concentration within the gas recirculation circuit (12).

9. A surgical gas delivery (10) system as recited in Claim 8, wherein a pump (24) is operatively associated with the gas recirculation circuit (12) to recirculate insufflation gas therethrough.

10. A surgical gas delivery system (10) as recited in Claim 8, wherein the gas metering valve (14) is a solenoid valve.

11. A surgical gas delivery system (10) as recited in Claim 8, wherein the gas metering valve (14) is a proportional valve.

**12.** A surgical gas delivery system (10) as recited in Claim 8, wherein the optical oxygen sensor (20) is adapted and configured to quantify a partial pressure of oxygen in the gas recirculation circuit (12) by measuring an infra-red wavelength of light absorption from a phosphor containing element and converting that measurement to a digital signal.

**Patentansprüche**

**1.** Chirurgisches Gaszuführsystem (10), umfassend:

a) einen Gasrückführungskreislauf (12) für Insufflationsgas;
b) einen optischen Sauerstoffsensor (20) zum Überwachen einer Insufflationsgaskonzentration;
c) ein Gasdosierventil (14) zum Dosieren eines Insufflationsgasstroms von einer Gasquelle (16) in den Gasrückführungskreislauf (12); und
d) einen Prozessor (18) zum Steuern des Gasdosierventils (14) zumindest teilweise auf der Basis von Signalen, die vom optischen Sauerstoffsensor (20) und einem Drucksensor (22) empfangen werden, um die Insufflationsgaskonzentration innerhalb des Gasrückführungskreislaufs (12) einzustellen.

**2.** Chirurgisches Gaszuführsystem (10) nach Anspruch 1, wobei eine Pumpe (24) funktionsfähig mit dem Gasrückführungskreislauf (12) verbunden ist, um Insufflationsgas durch diesen zurückzuführen.

**3.** Chirurgisches Gaszuführsystem (10) nach Anspruch 1, wobei das Gasdosierventil (14) ein Magnetventil ist.

**4.** Chirurgisches Gaszuführsystem (10) nach Anspruch 1, wobei das Gasdosierventil (14) ein Proportionalventil ist.

**5.** Chirurgisches Gaszuführsystem (10) nach Anspruch 1, wobei der Drucksensor (22) angepasst und eingerichtet ist, um den Insufflationsgasdruck innerhalb des Gasrückführungskreislaufs (12) zu messen.

**6.** Chirurgisches Gaszuführsystem (10) nach Anspruch 1, wobei der optische Sauerstoffsensor (20) angepasst und eingerichtet ist, um einen Sauerstoffpartialdruck im Gasrückführungskreislauf (12) zu quantifizieren.

**7.** Chirurgisches Gaszuführsystem (10) nach Anspruch 1, wobei der optische Sauerstoffsensor (20) angepasst und eingerichtet ist, um einen Sauerstoffpartialdruck im Gasrückführungskreislauf (12) zu quantifizieren, indem er eine Infrarotwellenlänge der Lichtabsorption von einem phosphorhaltigen Element misst und diese Messung in ein digitales Signal umwandelt.

**8.** Chirurgisches Gaszuführsystem (10) nach Anspruch 1 mit einem System zum Einstellen einer Insufflationsgaskonzentration im Gasrückführungskreislauf (12), wobei das System zum Einstellen einer Insufflationsgaskonzentration im Gasrückführungskreislauf (12) aufweist:

b) den optischen Sauerstoffsensor (20), der zum optischen Quantifizieren eines Sauerstoffpartialdrucks im Gasrückführungskreislauf (12) eingerichtet ist;
c) den Drucksensor (22), der zum Messen eines Insufflationsgasdrucks im Gasrückführungskreislauf (12) geeignet ist; und
d) den Prozessor (18), der geeignet ist, das Gasdosierventil (14) zumindest teilweise auf der Basis der Sauerstoffpartialdrucksignale im Gasrückführungskreislauf (12) und der Insufflationsgasdrucksignale im Gasrückführungskreislauf (12) zu steuern, um die Insufflationsgaskonzentration im Gasrückführungskreislauf (12) einzustellen.

**9.** Chirurgisches Gaszuführsystem (10) nach Anspruch 8, wobei eine Pumpe (24) funktionsfähig mit dem Gasrückführungskreislauf (12) verbunden ist, um das Insufflationsgas durch diesen zurückzuführen.

**10.** Chirurgisches Gaszuführsystem (10) nach Anspruch 8, wobei das Gasdosierventil (14) ein Magnetventil ist.

**11.** Chirurgisches Gaszuführsystem (10) nach Anspruch 8, wobei das Gasdosierventil (14) ein Proportionalventil ist.

**12.** Chirurgisches Gaszuführsystem (10) nach Anspruch 8, wobei der optische Sauerstoffsensor (20) angepasst und eingerichtet ist, um den Sauerstoffpartialdruck im Gasrückführungskreislauf (12) zu quantifizieren, indem er die Absorption von Licht im Infrarotbereich durch ein phosphorhaltiges Element misst und diese Messung in ein digitales Signal umwandelt.

**Revendications**

**1.** Système de distribution de gaz chirurgical (10) comprenant :

a) un circuit de recirculation de gaz (12) pour le gaz d'insufflation ;

b) un capteur optique d'oxygène (20) pour surveiller la concentration de gaz d'insufflation ;

c) une valve de dosage de gaz (14) pour doser un flux de gaz d'insufflation provenant d'une source de gaz (16) dans le circuit de recirculation de gaz (12) ; et

d) un processeur (18) pour commander la valve de dosage de gaz (14) sur la base, au moins en partie, des signaux reçus du capteur optique d'oxygène (20) et d'un capteur de pression (22) afin d'ajuster la concentration de gaz d'insufflation à l'intérieur du circuit de recirculation de gaz (12).

2. Système de distribution de gaz chirurgical (10) selon la revendication 1, dans lequel une pompe (24) est associée de manière fonctionnelle au circuit de recirculation de gaz (12) pour faire recirculer le gaz d'insufflation à travers celui-ci.

3. Système de distribution de gaz chirurgical (10) selon la revendication 1, dans lequel la valve de dosage de gaz (14) est une électrovalve.

4. Système de distribution de gaz chirurgical (10) selon la revendication 1, dans lequel la valve de dosage de gaz (14) est une valve proportionnelle.

5. Système de distribution de gaz chirurgical (10) selon la revendication 1, dans lequel le capteur de pression (22) est adapté et configuré pour mesurer la pression du gaz d'insufflation à l'intérieur du circuit de recirculation de gaz (12).

6. Système de distribution de gaz chirurgical (10) selon la revendication 1, dans lequel le capteur optique d'oxygène (20) est adapté et configuré pour quantifier une pression partielle d'oxygène dans le circuit de recirculation de gaz (12).

7. Système de distribution de gaz chirurgical (10) selon la revendication 1, dans lequel le capteur optique d'oxygène (20) est adapté et configuré pour quantifier une pression partielle d'oxygène dans le circuit de recirculation de gaz (12) en mesurant une longueur d'onde infrarouge d'absorption de lumière provenant d'un élément contenant du phosphore et en convertissant cette mesure en un signal numérique.

8. Système de distribution de gaz chirurgical (10) selon la revendication 1, comprenant un système pour ajuster la concentration de gaz d'insufflation à l'intérieur du circuit de recirculation de gaz (12), dans lequel ledit système pour ajuster la concentration de gaz d'insufflation à l'intérieur du circuit de recirculation de gaz (12) comprend :

b) le capteur optique d'oxygène (20) qui est configuré pour quantifier optiquement une pression partielle d'oxygène dans le circuit de recirculation de gaz (12) ;

c) le capteur de pression (22) qui est approprié pour mesurer la pression de gaz d'insufflation dans le circuit de recirculation de gaz (12) ; et

d) le processeur (18) qui est approprié pour commander la valve de dosage de gaz (14) sur la base, au moins en partie, des signaux de pression partielle d'oxygène dans le circuit de recirculation de gaz (12) et de pression de gaz d'insufflation dans le circuit de recirculation de gaz (12) afin d'ajuster la concentration de gaz d'insufflation à l'intérieur du circuit de recirculation de gaz (12).

9. Système de distribution de gaz chirurgical (10) selon la revendication 8, dans lequel une pompe (24) est associée de manière fonctionnelle au circuit de recirculation de gaz (12) pour faire recirculer le gaz d'insufflation à travers celui-ci.

10. Système de distribution de gaz chirurgical (10) selon la revendication 8, dans lequel la valve de dosage de gaz (14) est une électrovalve.

11. Système de distribution de gaz chirurgical (10) selon la revendication 8, dans lequel la valve de dosage de gaz (14) est une valve proportionnelle.

12. Système de distribution de gaz chirurgical (10) selon la revendication 8, dans lequel le capteur optique d'oxygène (20) est adapté et configuré pour quantifier une pression partielle d'oxygène dans le circuit de recirculation de gaz (12) en mesurant une longueur d'onde infrarouge d'absorption de lumière provenant d'un élément contenant du phosphore et en convertissant cette mesure en un signal numérique.

**Fig. 1**

**Fig. 2**

EP 4 558 201 B1

**Fig. 3**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2022117629 A1
- US 2019365414 A1
- US 2014336567 A1
- WO 2018039239 A1
- US 2018221598 A1
- US 9950127 B
- US 8795223 B
- US 9067030 B
- US 2022233794
- US 10495619 B